# EUROPEAN PATENT APPLICATION

(11) **EP 3 998 354 A1**
(43) Date of publication of application: **18.05.2022**
(21) Application number: 20837012.2
(22) Date of filing: 08.06.2020
(51) Int. Cl.: C12Q 1/6886, C12N 15/11

(54) **GENETIC MARKER COMBINATION AND APPLICATION THEREOF**

(30) Priority: 11.07.2019 CN 201910624566
(71) Applicant: Creative Biosciences (Guangzhou) Co., Ltd., Guangzhou, Guangdong 510530 (CN)
(72) Inventor: WU, Xiaolin, Guangzhou, Guangdong 510530 (CN); LIU, Xianglin, Guangzhou, Guangdong 510530 (CN); LUO, Yin, Guangzhou, Guangdong 510530 (CN); ZOU, Hongzhi, Guangzhou, Guangdong 510530 (CN)
(74) Representative: J A Kemp LLP
(86) International application number: PCT/CN2020/094954
(87) International publication number: WO 2021/004214

(57) **Abstract**

The present invention relates to a tumor marker, a methylation detection reagent, a kit and use thereof. By detecting the methylation level of SDC2, COL4A1/COL4A2 and ITGA4 genes, colorectal cancer specimens can be well distinguished from fecal specimens, and the detection sensitivity and specificity for the colorectal cancer can be 90% or above.

SEQUENCE LISTING

<110>  CREATIVE BIOSCIENCES (GUANGZHOU) CO., LTD.

<120>  Genetic Marker Combination and Application Thereof

<130>  N423665EP

<140>  EP 20837012.2
<141>  2020-06-08

<150>  CN201910624566
<151>  2019-07-11

<160>  17

<170>  PatentIn version 3.5

<210>  1
<211>  33
<212>  DNA
<213>  Artificial Sequence

<220>
<223>  capture sequence of SDC2 1

<400>  1
agcccgcgca cacgaatccg gagcagagta ccg                                    33


<210>  2
<211>  28
<212>  DNA
<213>  Artificial Sequence

<220>
<223>  capture sequence of SDC2 2

<400>  2
ctcctgccca gcgctcggcg cagcccgc                                          28


<210>  3
<211>  18
<212>  DNA
<213>  Artificial Sequence

<220>
<223>  SDC2-FP

<400>  3
gaggaagcga gcgttttc                                                     18


<210>  4
<211>  21
<212>  DNA
<213>  Artificial Sequence

<220>
<223>  SDC2-RP

<400>  4

aaaataccgc aacgattacg a 21

<210> 5
<211> 27
<212> DNA
<213> Artificial Sequence

<220>
<223> SDC2-Probe

<400> 5
agtttcgagt tcgagttttc gagtttg 27

<210> 6
<211> 35
<212> DNA
<213> Artificial Sequence

<220>
<223> capture sequence of COL4A1/COL4A2

<400> 6
gctgctgccc gaacgcattg gcccttccag aagca 35

<210> 7
<211> 22
<212> DNA
<213> Artificial Sequence

<220>
<223> COL4A1/COL4A2-FP

<400> 7
agagagttta gtaaggtcgg gc 22

<210> 8
<211> 21
<212> DNA
<213> Artificial Sequence

<220>
<223> COL4A1/COL4A2-RP

<400> 8
gacttcaaaa actactaccc g 21

<210> 9
<211> 18
<212> DNA
<213> Artificial Sequence

<220>
<223> COL4A1/COL4A2-Probe

<400> 9
tgtcggtgtg tcgtcggc 18

<210>  10
<211>  33
<212>  DNA
<213>  Artificial Sequence

<220>
<223>  capture sequnce of ITGA4

<400>  10
ctacgcgcgg ctgcaggggg cgctggggaa cct                                    33


<210>  11
<211>  19
<212>  DNA
<213>  Artificial Sequence

<220>
<223>  ITGA4-FP

<400>  11
acgcgagttt tgcgtagac                                                    19


<210>  12
<211>  20
<212>  DNA
<213>  Artificial Sequence

<220>
<223>  ITGA4-RP

<400>  12
gctaaataaa atcccgaacg                                                   20


<210>  13
<211>  23
<212>  DNA
<213>  Artificial Sequence

<220>
<223>  ITGA4-Probe

<400>  13
acggagttcg gttttgcgtt ttc                                               23


<210>  14
<211>  22
<212>  DNA
<213>  Artificial Sequence

<220>
<223>  COL4A1/COL4A2-FP

<400>  14
agagagttta gtaaggtcgg ac                                                22


<210>  15
<211>  21
<212>  DNA

<213>  Artificial Sequence

<220>
<223>  COL4A1/COL4A2-RP

<400>  15
gacttcaaaa actactaccc g                                                21


<210>  16
<211>  19
<212>  DNA
<213>  Artificial Sequence

<220>
<223>  ITGA4-RP

<400>  16
acgcgagttt tgcgtagtc                                                   19


<210>  17
<211>  20
<212>  DNA
<213>  Artificial Sequence

<220>
<223>  ITGA4-RP

<400>  17
gctaaataaa atcccgaacg                                                  20

## Description

### Technical Field

The present disclosure belongs to the field of biological medicine, in particular to a tumor marker, a methylation detection reagent, a kit and use thereof.

### Background Art

Colorectal cancer, also referred to as colon cancer, is a common malignant tumor of digestive tract. In China, the incidence rate of colorectal cancer is increasing year by year. In some coastal areas, such as Shanghai and Guangzhou, the incidence rate of colon cancer has ranked at the second place, second only to lung cancer. At present, it is believed that the intestinal cancer results from the accumulation of genetic defects and epigenetic defects. The early onset of colorectal cancer is hidden, often without obvious symptoms, and there may be symptoms such as hematochezia, abdominal pain, and diarrhea in the terminal stage. However, the appearance of symptoms usually indicates advanced cases, which brings great pain and expensive treatment cost to patients. Therefore, early detection, early diagnosis, and early treatment are important measures to reduce the incidence rate and mortality of colorectal cancer.

With screening, intestinal cancer and precancerous lesions can be detected early, and the lesions can be removed, thereby preventing the occurrence of intestinal cancer. The screening methods currently used for colorectal cancer mainly include occult blood test and colonoscopy. The occult blood test is susceptible to food or is not high in detection rate of adenoma. Although the colonoscopy is the gold standard for intestinal cancer diagnosis, it has low population compliance when used as a screening method. Therefore, there is an urgent need for a colorectal cancer screening method with high accuracy and high compliance.

Fecal gene detection, as a new intestinal cancer screening method, is now receiving more and more attention. This method (Cologuard^{®}) was included in the U.S. intestinal cancer screening guidelines in 2016. This method has the characteristics of convenience, noninvasion, and high detection rate for intestinal cancer and precancerous lesion adenomas. To prepare a fecal gene detection kit with high performance for colorectal cancer detection, two major obstacles need to be overcome: fecal DNA extraction and marker selection. On the one hand, the feces has complex compositions, many inhibitors to downstream reaction, and many bacterial DNAs. To extract human target genes from such a mixture, a set of highly sensitive gene extraction and purification methods is required. On the other hand, there are many markers related to intestinal cancer currently, especially DNA methylation markers, because studies have shown that DNA methylation is an early event of tumor formation. However, many methylation markers have good performance at the cell and tissue level, but they have reduced sensitivity and specificity for intestinal cancer when used in screening media such as feces and blood. For example, the sensitivity of a vimentin gene is 83% in tissues, but drops to 46% in fecal specimens (J Natl Cancer Inst. 2005 Aug 3; 97(15): 1124-32.).

In addition, due to complicated pathogenesis, colon cancer is a polygenic genetic disease. The existing methods for detection of single methylation markers for colon cancer have the following two limitations: First, the existing research and product detection methods both detect one marker in one PCR reaction well, and the detection method cannot realize multiple detection of methylation markers in one PCR reaction well. Second, when existing methylation markers for colon cancer detection, such as SDC2, ITAG4, and Septin 9, detect colon cancer by detecting the methylation level of a single gene, the sensitivity of detection is limited by genetic factors. The methylated SDC2 gene has a detection sensitivity of 84.2%, and a specificity of 97.9%; the methylated ITGA4 has a detection sensitivity of 83.8%, and a specificity of 95.2%; and the methylated Septin9 has a detection sensitivity of 79.3%, and a specificity of 94.3%. The specificity of these single markers for colon cancer detection can reach 90% or above, but when the specificity reaches 90% or above, the sensitivity cannot reach 90% or above.

Although a single methylation marker of colon cancer can detect part of colon cancer, it needs to combine related markers of colon cancer to detect as many colon cancer patients as possible through molecular biology methods. However, the problems that need to be faced are: 1. Most colon cancer gene methylation markers have overlapped performance in terms of detection value for colon cancer, i.e., the detection of a same colon cancer patient sample is consistent in most methylation markers. Therefore, it is very difficult to screen gene methylation markers that can complement each other in detection ability of colon cancer, and a great deal of researches are required. In addition, more gene methylation markers cause the superposition of false positive phenomena of the detection system, and the specificity of the detection will be reduced. Therefore, it is very difficult to improve the sensitivity of colon cancer detection under the premise of certain detection specificity. 2. The detection cost, sensitivity and specificity need to be balanced. Chinese patent application CN109207592A analyzes a model of detecting colorectal cancer with multiple markers. The sensitivity of the combined detection of SEPT9, NDRG4, and SDC2 is only 88.6%, and the specificity is only 89.1%; in a variety of different combination detection models of KRAS, BMP3, NDRG4, SEPT9, and SDC2, the highest AUC value of the ROC curve is only 0.912, and it requires simultaneous combined detection of 5 genetic markers to obtain this 0.912 AUC.

### Summary of the Invention

In some embodiments, a gene marker for colorectal cancer with high specificity and high sensitivity, and a diagnostic reagent for the gene marker and use thereof are provided.

In some embodiments, provided is a gene marker combination, including SDC2, COL4A1/COL4A2 and ITGA4. Compared with existing markers for intestinal cancer detection, the SDC2, COL4A1/COL4A2 and ITGA4 gene combination and technical solution provided in the present disclosure can detect colorectal cancer with extremely high sensitivity and specificity. The sensitivity and specificity of the technical solution of the present disclosure for colorectal cancer detection are both higher than 90%.

In some embodiments, the combined detection of methylation of SDC2, COL4A1/COL4A2 and ITGA4 gene combination can realize the combined detection of multiple genes, which greatly reduces the complexity of test and improves the detection efficiency.

In some embodiments, provided is a detection reagent/detection kit, including a reagent for methylation detection of SDC2, COL4A1/COL4A2 and ITGA4 genes.

In some embodiments, as for the methylation detection reagent containing the SDC2, COL4A1/COL4A2 and ITGA4 gene combination, the extraction detection method can very conveniently and accurately determine colorectal cancer and normal people. The methylation detection reagent of the gene combination is expected to be used for fecal gene detection kits, and serve for the clinical detection of intestinal cancer.

In some embodiments, the reagent/kit detects and diagnoses cancer by methylation level. More and more studies have confirmed that methylation change is an early event in the process of tumorigenesis, and it is easier to find early lesions by detecting abnormal methylation.

In some embodiments, the detection reagent/detection kit includes a capture sequence, a primer and/or a probe for the SDC2, COL4A1/COL4A2 and ITGA4 genes.

In some embodiments, the capture sequence is an isolated capture sequence.

In some embodiments, the primer is an isolated primer.

In some embodiments, the probe is an isolated probe.

In some embodiments, the detection reagent/detection kit includes a capture sequence, a primer and/or a probe obtainable for CpG island of each gene.

In some embodiments, the capture sequence for methylation detection of the SDC2 gene includes any one of nucleotide sequences as shown below:
I. a nucleotide sequence having at least 85% or at least 90% or at least 91% or at least 92% or at least 93% or at least 94% or at least 95% or at least 96% or at least 97% or at least 98% or at least 99%, or 100% identity to the sequences as set forth in any of SEQ ID NO:1 and 2; and
II. a sequence complementary to the sequence as set forth in I; and/or
   the capture sequence for methylation detection of the COL4A1/COL4A2 gene includes any one of nucleotide sequences as shown below:
   III. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:6; and
IV. a sequence complementary to the sequence as set forth in III; and/or
   the capture sequence for methylation detection of the ITGA4 gene includes any one of nucleotide sequences as shown below:
   V. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:10; and
VI. a sequence complementary to the sequence as set forth in V.
   In some embodiments, the upstream primer in the primer for methylation detection of the SDC2 gene includes any one of nucleotide sequences as shown below:
   VII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:3; and
VIII. a sequence complementary to the sequence as set forth in VII; and/or
   the downstream primer in the primer for methylation detection of the SDC2 gene includes any one of nucleotide sequences as shown below:
   IX. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:4; and
X. a sequence complementary to the sequence as set forth in IX; and/or
   the upstream primer in the primer for methylation detection of the COL4A1/COL4A2 gene includes any one of nucleotide sequences as shown below:
   XI. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:7 and 18; and
XII. a sequence complementary to the sequence as set forth in XI; and/or
   the downstream primer in the primer for methylation detection of the COL4A1/COL4A2 gene includes any one of nucleotide sequences as shown below:
   XIII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:8 and 19; and XIV. a sequence complementary to the sequence as set forth in XIII.

In some embodiments, the primer pair for methylation detection of the COL4A1/COL4A2 gene is as set forth in SEQ ID NO:7 and SEQ ID NO:8.

In some embodiments, the primer pair for methylation detection of the COL4A1/COL4A2 gene is as set forth in SEQ ID NO:18 and SEQ ID NO:19; and/or
the upstream primer in the primer for methylation detection of the ITGA4 gene includes any one of nucleotide sequences as shown below:
XV. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:11 and SEQ ID NO:16; and
XVI. a sequence complementary to the sequence as set forth in XV; and/or
   the downstream primer in the primer for methylation detection of the ITGA4 gene includes any one of nucleotide sequences as shown below:
   XVII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:12 and SEQ ID NO:17; and
XVIII. a sequence complementary to the sequence as set forth in XVII.

In some embodiments, the primer pair for methylation detection of the ITGA4 gene is as set forth in SEQ ID NO:11 and SEQ ID NO:12.

In some embodiments, the primer pair for methylation detection of the ITGA4 gene is as set forth in SEQ ID NO:16 and SEQ ID NO:17.

In some embodiments, the probe for methylation detection of the SDC2 gene includes any one of nucleotide sequences as shown below:
XIX. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:5; and
XX. a sequence complementary to the sequence as set forth in XIX; and/or the probe for methylation detection of the COL4A1/COL4A2 gene includes any one of nucleotide sequences as shown below:
   XXI. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:9; and
XXII. a sequence complementary to the sequence as set forth in XXI; and/or
   the probe for methylation detection of the ITGA4 gene includes any one of nucleotide sequences as shown below:
   XXIII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:13; and
XXIV. a sequence complementary to the sequence as set forth in XXIII.

In some specific embodiments, by detecting the methylation level of the promoter region of each gene of the SDC2, COL4A1/COL4A2 and ITGA4 gene combination, colorectal cancer specimens can be well distinguished from fecal specimens. Colorectal cancer is detected by using the methylation combination detection reagent containing the gene combination, and the detection sensitivity and specificity for colorectal cancer are extremely high.

In some embodiments, provided is use of a combined detection reagent for methylation of multiple genes in preparation of a colorectal tumor detection reagent or kit, and the genes include SDC2, COL4A1/COL4A2 and ITGA4.

In some embodiments, the combined detection reagent for methylation of multiple genes includes a capture sequence, a primer and/or a probe for methylation detection of each gene.

In some embodiments, the reagent includes a capture sequence, a primer and/or a probe obtainable for the CpG island of each gene.

The "COL4A1/COL4A2" in the present disclosure refers to "COL4A1 or COL4A2". The human genes COL4A1 and COL4A2 are closely linked at the distal end of the long arm of chromosome 13. These two genes are in a "head-to-head" positional relationship, and are opposite in transcription direction. The 5' ends of the COL4A1 and COL4A2 genes are close to each other with a gap of 127bp. This sequence is a bidirectional promoter region shared by the two genes [1]. Therefore, the sequence designed according to the bidirectional promoter shared by the two can target both COL4A1 and COL4A2. In the present disclosure, there is no difference in labeling sequence information with COL4A1 or COL4A2.

The gene markers SDC2, COL4A1/COL4A2, and ITGA4 in the present disclosure are combinedly detected. In some embodiments, SDC2, COL4A1/COL4A2, and ITGA4 can be detected separately for methylation levels. In other embodiments, SDC2, COL4A1/COL4A2, and ITGA4 can be simultaneously detected for methylation levels. For example, the methylation levels of multiple genes are detected by multiplex real-time fluorescent quantitative PCR. In certain aspects, it is more convenient for detecting SDC2, COL4A1/COL4A2 and ITGA4 simultaneously by multiplex PCR.

In the present disclosure, "detection" is equivalent to "diagnosis", which includes the diagnosis of colorectal tumors in the middle and terminal stages in addition to the early diagnosis of colorectal tumors, and also includes colorectal tumor screening, risk assessment, prognosis, disease identification, diagnosis of disease stages, and selection of therapeutic targets.

The use of the colorectal tumor marker combination of SDC2, COL4A1/COL4A2 and ITGA4 makes the early diagnosis of colorectal tumor possible. When it is determined that a gene methylated in a cancer cell is methylated in a clinically or morphologically normal cell, it indicates that the normal cell is developing into cancer. In this way, colorectal cancer can be diagnosed at an early stage by the methylation of the colorectal tumor-specific SDC2, COL4A1/COL4A2, and ITGA4 gene combination in normal cells.

The early diagnosis refers to the possibility of detecting cancer before metastasis, preferably before the morphological changes of tissues or cells are observable.

In addition to the early diagnosis of colorectal tumors, the reagents/kits of the present disclosure are also promising for colorectal tumor screening, risk assessment, prognostic diagnosis, disease identification, diagnosis of disease stages, and selection of therapeutic targets.

As an alternative implementation of the disease stage, the progression of colorectal tumors at different stages or periods can be diagnosed by measuring the methylation degree of the SDC2, COL4A1/COL4A2 and ITGA4 gene combination obtained from samples. By comparing the methylation degree of the SDC2, COL4A1/COL4A2, and ITGA4 gene combination of nucleic acids isolated from samples of each stage of colorectal cancer with the methylation degree of the SDC2, COL4A1/COL4A2 and ITGA4 gene combination of one or more nucleic acids isolated from a sample of intestinal tissues without abnormal cell proliferation, the specific stage of colorectal tumor in the sample can be detected.

Generally, the CpG island refers to some regions rich in CpG dinucleotides, usually located in the promoter and its vicinity. The detection sites for methylation in the present disclosure include not only the CpG island, but also heterozygous methylated CpG sites in other regions such as genosome or intergenic regions, or an isolated CpG site.

The combined detection reagent for methylation of the SDC2, COL4A1/COL4A2 and ITGA4 gene combination may be a methylation detection reagent in the prior art. In the prior art, there are many methods that can detect the methylation of target genes, such as methylation-specific PCR (MSP), methylation-specific quantitative PCR (qMSP), methylation DNA specific binding protein PCR, quantitative PCR and DNA chips, methylation-sensitive restriction endonucleases, bisulfite sequencing or pyrosequencing. In addition, other methylation detection methods can be introduced through patent US62007687. Each detection method has its corresponding reagents, and all these reagents can be used in the present disclosure to detect the methylation of the SDC2, COL4A1/COL4A2 and ITGA4 gene combination.

In some embodiments, the present disclosure also provides the detection reagent/detection kit of the SDC2, COL4A1/COL4A2 and ITGA4 gene combination, including a capture sequence, a primer and/or a probe obtained for each gene in the SDC2, COL4A1/COL4A2 and ITGA4 gene combination.

In some specific embodiments of the present disclosure, a capture sequence, a primer and/or a probe obtainable for the CpG island of each gene of the SDC2, COL4A1/COL4A2 and ITGA4 gene combination is included.

In some specific embodiments of the present disclosure, the primer and/or probe detects the methylation of each gene in the SDC2, COL4A1/COL4A2 and ITGA4 gene combination by quantitative Methylation-Specific PCR (qMSP).

In some specific embodiments of the present disclosure, the methylation detection reagent provided by the present disclosure detects the methylation level of the genosome, intergenic region or promoter region and the vicinity of the promoter region of each gene in the SDC2, COL4A1/COL4A2 and ITGA4 gene combination.

In some specific embodiments of the present disclosure, the methylation detection reagent provided by the present disclosure includes a capture sequence, a primer and/or a probe obtained for the CpG island of the promoter region or the vicinity of the promoter region of each gene in the SDC2, COL4A1/COL4A2 and ITGA4 gene combination.

In some specific embodiments of the present disclosure, the capture sequence of the SDC2 gene in the combined detection reagent for methylation provided by the present disclosure includes any one of nucleotide sequences as shown below:
I. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:1 and 2; and
II. a sequence complementary to the sequence as set forth in I; and/or
   the capture sequence for methylation detection of the COL4A1/COL4A2 gene includes any one of nucleotide sequences as shown below:
   III. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:6; and
IV. a sequence complementary to the sequence as set forth in III; and/or
   the capture sequence for methylation detection of the ITGA4 gene includes any one of nucleotide sequences as shown below:
   V. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:10; and
VI. a sequence complementary to the sequence as set forth in V.

In some specific embodiments of the present disclosure, the upstream primer in the primer for methylation detection of the SDC2 gene includes any one of nucleotide sequences as shown below:
VII. a nucleotide sequence having at least 85% or at least 90% or at least 91% or at least 92% or at least 93% or at least 94% or at least 95% or at least 96% or at least 97% or 98% or at least 99%, or 100% identity to has the sequence as set forth in SEQ ID NO:3; and
VIII. a sequence complementary to the sequence as set forth in VII; and/or
   the downstream primer in the primer for methylation detection of the SDC2 gene includes any one of nucleotide sequences as shown below:
   IX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:4; and
X. a sequence complementary to the sequence as set forth in IX; and/or the upstream primer in the primer for methylation detection of the COL4A1/COL4A2 gene includes any one of nucleotide sequences as shown below:
   XI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:7 and 14; and
XII. a sequence complementary to the sequence as set forth in XI; and/or
   the downstream primer in the primer for methylation detection of the COL4A1/COL4A2 gene includes any one of nucleotide sequences as shown below:
   XIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:8 and 15; and
XIV. a sequence complementary to the sequence as set forth in XIII.

In some specific embodiments of the present disclosure, the primer pair for methylation detection of the COL4A1/COL4A2 gene is as set forth in SEQ ID NO:7 and SEQ ID NO:8.

In some specific embodiments of the present disclosure, the primer pair for methylation detection of the COL4A1/COL4A2 gene is as set forth in SEQ ID NO:14 and SEQ ID NO:15.

In some embodiments, the upstream primer in the primer for methylation detection of the ITGA4 gene includes any one of nucleotide sequences as shown below:
XV. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:11 and SEQ ID NO:16; and
XVI. a sequence complementary to the sequence as set forth in XV; and/or
   the downstream primer in the primer for methylation detection of the ITGA4 gene includes any one of nucleotide sequences as shown below:
   XVII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:12 and SEQ ID NO:17; and
XVIII. a sequence complementary to the sequence as set forth in XVII.

In some specific embodiments of the present disclosure, the primer pair for methylation detection of the ITGA4 gene is as set forth in SEQ ID NO:11 and SEQ ID NO:12.

In some specific embodiments of the present disclosure, the primer pair for methylation detection of the ITGA4 gene is as set forth in SEQ ID NO:16 and SEQ ID NO:17.

In some specific embodiments of the present disclosure, the probe for methylation detection of the SDC2 gene includes any one of nucleotide sequences as shown below:
XIX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:5; and
XX. a sequence complementary to the sequence as set forth in XIX; and/or the probe for methylation detection of the COL4A1/COL4A2 gene includes any one of nucleotide sequences as shown below:
   XXI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:9; and
XXII. a sequence complementary to the sequence as set forth in XXI; and/or the probe for methylation detection of the ITGA4 gene includes any one of nucleotide sequences as shown below:
   XXIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:13; and
XXIV. a sequence complementary to the sequence as set forth in XXIII.

In some embodiments, the nucleic acid probe also contains labels such as one or more of a radioisotope, a fluorescent group, a bioluminescent compound, a chemiluminescent compound, a metal chelate and an enzyme.

In some embodiments, the labels of the probe are fluorescent groups, and these fluorescent groups include but are not limited to one or more of VIC, ROX, FAM, Cy5, HEX, TET, JOE, NED and

### Texas Red.

In some embodiments, the probes of SDC2, COL4A1/COL4A2 and ITGA4 genes are labeled with a same fluorescent group, such that the sum of methylation levels of these genes can be easily detected through one fluorescent channel, without the need of detecting each gene by using different fluorescence channels, which reduces the complexity of test.

In some embodiments, the present disclosure also provides a kit for tumor detection, including the combined detection reagent for methylation.

In some specific embodiments of the present disclosure, the kit provided by the present disclosure includes: a first container, containing a capture reagent; a second container, containing a primer pair for amplification; and a third container, containing a probe.

In some specific embodiments of the present disclosure, the kit provided by the present disclosure also includes reagents commonly used in the kit, such as a conversion agent commonly used in qMSP, which is used to convert all unmethylated cytosine bases into uracil, while the methylated cytosine bases are remained. There is no particular limitation on the conversion agent. The reagents reported in the prior art that can convert cytosine to uracil can be used, such as one or several of hydrazine salt, bisulfite and hydrosulfite (such as sodium metabisulfite, potassium bisulfite, cesium bisulfite, ammonium bisulfite). For another example, the kit also includes DNA polymerases, dNTPs, Mg²⁺ ions, buffers, and the like commonly used in amplifying the COL4A1 gene.

In some specific implementations, the kit includes: a first container, containing a capture reagent; a second container, containing a primer pair for amplification; a third container, containing a probe; and a fourth container, containing a conversion reagent for converting unmethylated cytosine.

In some specific implementations, the kit also includes instructions.

In some specific implementations, the kit also includes a nucleic acid extraction reagent.

In some specific implementations, the kit also includes a sampling device.

In some embodiments, the present disclosure provides use of a combined detection reagent for methylation of SDC2, COL4A1/COL4A2 and ITGA4 genes in preparation of a colorectal tumor detection reagent or kit.

In some embodiments, the present disclosure provides a primer, which is selected from at least one of sequences having at least 85% or at least 90% or at least 91% or at least 92% or at least 93 % or at least 94% or at least 95% or at least 96% or at least 97% or 98% or at least 99%, or 100% identity to the sequences as set forth in SEQ ID NO: 3, SEQ ID NO: 4, SEQ ID NO: 7, SEQ ID NO: 8, SEQ ID NO: 11, and SEQ ID NO: 12., SEQ ID NO:14, SEQ ID NO:15, SEQ ID NO:16, SEQ ID NO:17 or complementary sequences thereof.

In some embodiments, the primer is selected from any one of the sequences as set forth in SEQ ID NO: 3, SEQ ID NO: 4, SEQ ID NO: 7, SEQ ID NO: 8, SEQ ID NO: 11, SEQ ID NO: 12, SEQ ID NO: 14, SEQ ID NO: 15, SEQ ID NO: 16, and SEQ ID NO: 17.

In some embodiments, the primers are selected from at least one primer pair of a sequence having at least 85% or at least 90% or at least 91% or at least 92% or at least 93% or at least 94% or at least 95% or at least 96 % or at least 97% or 98% or at least 99%, or 100% identity to the sequences as set forth in SEQ ID NO: 3 and SEQ ID NO: 4, SEQ ID NO: 7 and SEQ ID NO: 8, SEQ ID NO: 11 and SEQ ID NO: 12, SEQ ID NO: 14 and SEQ ID NO: 15 and SEQ ID NO: 16 and SEQ ID NO: 17.

In some embodiments, the primer is selected from the primer pairs as set forth in SEQ ID NO: 3 and SEQ ID NO: 4, SEQ ID NO: 7 and SEQ ID NO: 8, and SEQ ID NO: 11 and SEQ ID NO: 12. In some embodiments, the primers are selected from the primer pairs as set forth in SEQ ID NO: 3 and SEQ ID NO: 4, SEQ ID NO: 14 and SEQ ID NO: 15, and SEQ ID NO: 16 and SEQ ID NO: 17.

In some embodiments, the present disclosure also provides a capture sequence, which is selected from at least one of sequences having 85% or at least 90% or at least 91% or at least 92% or at least 93% or at least 94% or at least 95% or at least 96% or at least 97% or 98% or at least 99%, or 100% identity to the sequences as set forth in SEQ ID NO: 1, SEQ ID NO: 2, SEQ ID NO: 6, SEQ ID NO: 10 or complementary sequences thereof.

In some embodiments, the capture sequence is selected from at least one of the sequences as set forth in SEQ ID NO: 1, SEQ ID NO: 2, SEQ ID NO: 6, and SEQ ID NO: 10.

In some embodiments, the capture sequence is selected from the sequences as set forth in SEQ ID NO:1, SEQ ID NO:6 and SEQ ID NO:10.

In some embodiments, the capture sequence is selected from the sequences as set forth in SEQ ID NO:2, SEQ ID NO:6 and SEQ ID NO:10.

In some embodiments, the present disclosure also provides a nucleic acid probe, which is selected from at least one of sequences having at least 85% or at least of 90% or at least 91% or at least 92% or at least 93% or at least 94% or at least 95% or at least 96% or at least 97% or 98% or at least 99%, or 100% identity to the sequences as set forth in SEQ ID NO: 5, SEQ ID NO: 9, SEQ ID NO: 13 or complementary sequences thereof.

In some embodiments, the nucleic acid probe is selected from at least one of SEQ ID NO: 5, SEQ ID NO: 9, and SEQ ID NO: 13.

In some embodiments, the nucleic acid probe is selected from the sequences as set forth in SEQ ID NO: 5, SEQ ID NO: 9 and SEQ ID NO: 13.

In some embodiments, the present disclosure also provides use of the abovementioned methylation detection reagent, kit, capture sequence, primer and/or probe in preparation of a reagent or kit for methylation detection, or in preparation of a reagent or kit for colorectal tumor detection.

In some embodiments, the present disclosure also provides use of the aforementioned methylation detection reagent, kit, capture sequence, primer and/or probe in methylation detection, or use in colorectal tumor detection.

In some embodiments, the present disclosure also provides a colorectal tumor detection system, including the following components:
(1) a combined detection component for methylation of SDC2, COL4A1/COL4A2 and ITGA4 genes;
(2) a data processing component; and
(3) a result output component.

In some specific embodiments of the present disclosure, the methylation detection component includes a methylation detection instrument.

In some specific embodiments of the present disclosure, the methylation detection component also includes one or more of the combined detection reagent for methylation, the capture sequence, the primer and the probe.

In some specific embodiments of the present disclosure, the methylation detection instrument includes one or more of a fluorescent quantitative PCR instrument, a PCR instrument, and a sequencer.

In some specific embodiments of the present disclosure, the data processing component includes a data processing instrument.

The data processing instrument includes any apparatus or instrument or device that can be used by those skilled in the art for data processing.

In some specific embodiments of the present disclosure, the data processing instrument includes one or more of a calculator and a computer.

The computer is loaded with any software or program that can be used by those skilled in the art for data processing or statistical analysis.

In some specific embodiments of the present disclosure, the computer includes a computer loaded with one or more software of SPSS, SAS, and Excel.

In some specific embodiments of the present disclosure, the result output component includes a result output device.

The output device includes any apparatus or instrument or device that can display the data processing result as readable content.

In some specific embodiments of the present disclosure, the result output device includes one or more of a screen and a paper report.

In some specific embodiments of the present disclosure, the data processor is configured to a. receive the test data of the test sample and the normal control sample; b. store the test data of a test sample and a normal control sample; c. compare the test data of the test sample with that of the normal control sample that is the same type; and d. respond to the probability or possibility that a subject will develop a tumor according to the comparison results.

In some specific embodiments of the present disclosure, the result output component is used to output the probability or possibility that a subject will develop a tumor.

In some embodiments, the present disclosure also provides a method for diagnosing a colorectal tumor, including the following steps of:
(1) detecting the methylation levels of the SDC2, COL4A1/COL4A2 and ITGA4 genes in a test sample derived from the subject;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample.

In some specific embodiments of the present disclosure, the methylation level detection of the SDC2, COL4A1/COL4A2 and ITGA4 genes adopts methylation quantitative detection methods in the prior art, such as quantitative methylation-specific PCR (qMSP).

In some specific embodiments of the present disclosure, the methylation level detection of the SDC2, COL4A1/COL4A2 and ITGA4 genes adopts the abovementioned capture sequence, primer and/or probe.

The diagnosing method of the present disclosure can be used before and after the treatment of colorectal tumors or in combination with the treatment. Use after the treatment includes, for example, evaluating the success of treatment or monitoring the remission, recurrence, and/or progression (including metastasis) of the colorectal tumor after treatment.

In some embodiments, the present disclosure provides a method for diagnosing a colorectal tumor, including the following steps of:
(1) detecting the methylation levels of the SDC2, COL4A1/COL4A2 and ITGA4 genes in a test sample derived from the subject; the detecting including contacting the test sample of the subject with a detection reagent for the methylation levels of SDC2, COL4A1/COL4A2 and ITGA4 genes;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample.

In some embodiments, the present disclosure provides a method for treating a colorectal tumor, including administering surgery, chemotherapy, radiotherapy, radiotherapy and chemotherapy, immunotherapy, oncolytic virus therapy, or any other types of colorectal tumor treatment methods used in the art and combinations of these treatment methods to patients diagnosed with colorectal tumors by the above diagnosing method.

In some embodiments, provided is a method for treating a colorectal tumor, including the following steps of:
(1) detecting the methylation levels of the SDC2, COL4A1/COL4A2 and ITGA4 genes in a test sample derived from the subject;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample; if the subject is diagnosed as a patient with colorectal tumor, administering to the subject a drug for treating the colorectal tumor.

In some specific embodiments of the present disclosure, the determination standard of the data processing component in the tumor detection system or the method for diagnosing colorectal tumor is to determine tumor specimens and normal specimens according to the cutoff of the Ct value of quantitative methylation-specific PCR (qMSP).

In some specific embodiments of the present disclosure, the cutoff of Ct value is about 36-39.

In some specific embodiments of the present disclosure, the cutoff of Ct value is about 38.

In some specific embodiments of the present disclosure, the cutoff of the above Ct value is about 36-39.

In some embodiments, the sample involved is a feces sample.

In the abovementioned another technical solution, the methylation detection reagent of the SDC2, COL4A1/COL4A2 and ITGA4 gene combination can detect 91.36% of colorectal cancer in fecal specimens when the specificity is 95.28%. Feces can be simply used as a test sample for reliable diagnosis of colorectal cancer. The feces sample is very easy to obtain, and the sampling is non-invasive and simple, without causing any pain and inconvenience to patients.

In some specific embodiments of the present disclosure, when the Ct value of the test sample is less than the cutoff of the Ct value, it is determined as the tumor sample, and when the Ct value of the test sample is greater than or equal to the cutoff of the Ct value, it is determined as the normal sample.

In some specific embodiments of the present disclosure, the tumor described in the present disclosure is a colorectal tumor.

In some specific embodiments of the present disclosure, the tumor described in the present disclosure is colorectal cancer or adenoma.

In some specific embodiments of the present disclosure, the test sample or sample type involved in the present disclosure includes tissue, body fluid, or excrement.

In some specific embodiments of the present disclosure, the tissue described in the present disclosure includes intestinal tissue.

In some specific embodiments of the present disclosure, the body fluid described in the present disclosure includes blood, serum, plasma, extracellular fluid, tissue fluid, lymphatic fluid, cerebrospinal fluid or aqueous humor.

In some specific embodiments of the present disclosure, the excrement includes sputum, urine, saliva or feces.

In some specific embodiments of the present disclosure, the excrement includes feces.

In some embodiments, the above sequence is an isolated sequence.

### Brief Description of the Drawings

FIG. 1 shows a ROC curve of SDC2, COL4A1/COL4A2 and ITG4 gene combined detection of colorectal cancer and adenoma (≥1 cm) of 935 fecal specimens in Example 1.
FIG. 2 shows detection results of fecal specimens before and after operation of 23 colorectal cancer patients detected by a method of SDC2, COL4A1/COL4A2 and ITG4 gene combined detection in Example 2.
FIG. 3 shows a ROC curve of SOX21 gene for colorectal cancer and adenoma detection of 240 fecal specimens in Comparative Example 1.
FIG. 4 shows a ROC curve of ITGA4, COL4A1/COL4A2 gene combined detection of colorectal cancer of 109 fecal specimens in Comparative Example 2.

### Detailed Description of the Invention

The technical solutions of the present disclosure are further described by specific examples below, and the specific examples do not pose a limitation on the protection scope of the present disclosure. Some non-essential modifications and adjustments made by others based on the concept of the present disclosure still fall within the protection scope of the present disclosure.

The "capture sequence", "primer" or "probe" in the present disclosure refers to an oligonucleotide that contains a region complementary to a sequence of at least 6 consecutive nucleotides of a target nucleic acid molecule (e.g., a target gene). In some embodiments, at least part of the sequence of the primer or probe is not complementary to an amplified sequence. In some embodiments, the primer or probe contains a region complementary to a sequence of at least 9, at least 10, at least 11, at least 12, at least 13, at least 14, at least 15, at least 16, at least 17, at least 18, at least 19, or at least 20 consecutive nucleotides of a target molecule. When a primer or probe contains a region "complementary to at least x consecutive nucleotides of a target molecule", the primer or probe is at least 95% complementary to at least x consecutive or discontinuous block nucleotides of the target molecule. In some embodiments, the primer or probe is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, 96%, at least 97%, at least 98%, at least 99%, or 100% complementary to the target molecule.

In the present disclosure, "normal" samples refer to samples of the same type isolated from individuals who are known to be free of the cancer or tumor.

In the present disclosure, the "subject" is a mammal, such as a human.

The samples for methylation detection in the present disclosure include but are not limited to DNAs, or RNAs, or DNA and RNA samples containing mRNAs, or DNA-RNA hybrids. The DNA or RNA may be single-stranded or double-stranded.

In the present disclosure, the "methylation level" is equivalent to "methylation degree", which can usually be expressed as the percentage of methylated cytosine, which is obtained through dividing the number of methylated cytosine by the sum of the number of methylated cytosine and the number of unmethylated cytosine; be expressed with the generally used method of dividing the number of methylation target genes by the number of internal reference genes; and be expressed with other methods of expressing the methylation level in the prior art.

In the present disclosure, the "sample" is equivalent to "specimen".

As used herein, the term "and/or" refers to and encompasses any and all possible combinations of one or more of associated listed items. When used in a list of two or more items, the term "and/or" means that any of the listed items can be used alone, or any combination of two or more of the listed items can be used. For example, if a composition, a combination, a structure, and the like are described as including (or comprising) components A, B, C and/or D, then the composition may include A alone; B alone; C alone; D alone; a combination of A and B; a combination of A and C; a combination of A and D; a combination of B and C; a combination of B and D; a combination of C and D; a combination of A, B and C; a combination of A, B and D; a combination of A, C and D; a combination of B, C and D; or a combination of A, B, C and D.

### Example 1

935 fecal specimens (359 cases of colorectal cancer, 67 cases of adenoma (≥1 cm), 509 cases of non-tumor individuals, all confirmed by colonoscopy or pathology) were selected for grinding and centrifugation, 50 ul of capture magnetic beads (containing capture sequences of SDC2, COL4A2, ITGA4 and reference gene ACTB) were added, and the operations were carried out according to the technical solution described below.

The technical solution was as follows:
1) Fecal specimens of normal people and colorectal tumor patients with colonoscopy pathology results were collected, mixed and ground according to 1 g of fences: 4 mL of protective solution, and then centrifuged at 5000 rpm for 10 min, a supernatant was taken and precipitates were discarded.
2) 10 mL of supernatant was taken and centrifuged again, and 3.2 mL of supernatant was taken, into which 2 mL of lysate and 50 ul of capture magnetic beads M1 were added, incubated at 95°C for 15 min, and then placed at room temperature for 30 min.
3) The mixture was placed on a magnetic stand, part of supernatant was discarded, the magnetic beads were washed off, the mixture was transferred to a 2 mL centrifuge tube, 800 ul of washing solution W1 was added, incubation was carried out at 1300 rpm for 1 min at room temperature, the mixture was placed on the magnetic stand to aspirate the supernatant, and this process was repeated 2 times;
4) 50 ul of eluent was added, incubated at room temperature at 1300 rpm for 5 minutes, and placed on a magnetic stand, and an eluent was transferred to a new EP tube in 3 minutes.
5) DNA fragments in the previous step was methylated by using an EZ DNA Methylation Kit (Zymo Research) according to the method in Reference [2], and 20 ul of final eluate was used for qMSP detection.

Finally, 20 ul of Bisulfite-transformed DNA was obtained. Then qMSP detection was carried out, and finally the methylation level of the SDC2, COL4A1/COL4A2 and ITG4 gene combination in the specimen was determined according to the CT value.

The qMSP reaction system of this example: 30 ul (nuclease-free water 2.98 ul, 5×Colorless GoTaq Flexi Buffer 6 ul, MgCl₂ (25 mM) 5 ul, dNTPs (10 mM) 1 ul, GoTaq Hot Start polymerase 0.6 ul, ACTB-FP (100 uM) 0.08 ul, ACTB-RP (100 uM) 0.08 ul, ACTB-Probe (100 uM) 0.06 ul, SDC2-FP (100 uM) 0.12 ul, SDC2-RP (100 uM) 0.12 ul, SDC2-Probe (100 uM) 0.04 ul, COL4A2-FP (100 uM) 0.06 ul, COL4A2-RP(100 uM) 0.06 ul, COL4A2-Probe(100 uM) 0.04 ul, ITGA4-FP (100 uM) 0.06 ul, ITGA4-RP(100 uM) 0.06 ul, ITGA4-Probe (100 uM) 0.04 ul, DNA 10 ul). Reaction procedure: 95°C 5 minutes, (95°C 15s, 58°C 30s, 72°C 30s) × 48 Cycles, 40°C 30s.

The capture and PCR reaction uses ACTB as the reference gene, and finally the methylation level in the specimen is determined according to the CT value. The target gene is determined as positive with a CT value ≤ 38, and as negative with a CT value> 38.

Because the 5' ends of the COL4A1 and COL4A2 genes are close to each other with a gap of 127bp, they share a bidirectional promoter region. The methylation region detected in this example is the bidirectional promoter region of the COL4A1 and COL4A2 genes. Therefore, in this example, the gene direction of COL4A2 of the two genes is selected to label sequence information.

The methylation sites of the SDC2, COL4A1/COL4A2 and ITGA4 genes are mainly located in the promoter region or CpG island nearby.

In this example, the PCR probes of SDC2, COL4A2 and ITGA4 are labeled with a same fluorescent group, so the sum of methylation levels of these genes can be easily detected through one fluorescent channel, without the need of detecting each gene by using different fluorescence channels, which reduces the complexity of test.

The capture sequences, primers and probes of this example are as follows:
SEQ ID NO.1: capture sequence 1 of SDC2:
   5'-AGCCCGCGCACACGAATCCGGAGCAGAGTACCG-3' or
SEQ ID NO.2: capture sequence 2 of SDC2:
   5'-CTCCTGCCCAGCGCTCGGCGCAGCCCGC-3'
qMSP primer and probe of SDC2:
   SEQ ID NO.3: SDC2-FP: 5'-GAGGAAGCGAGCGTTTTC-3'
   SEQ ID NO.4: SDC2-RP: 5'-AAAATACCGCAACGATTACGA-3'
   SEQ ID NO.5: SDC2-Probe: 5'-AGTTTCGAGTTCGAGTTTTCGAGTTTG-3'

The two capture sequences of SDC2 have the same capture effect, and either one can be selected.
SEQ ID NO.6: capture sequence of COL4A1/COL4A2:
   5'-GCTGCTGCCCGAACGCATTGGCCCTTCCAGAAGCA-3'
qMSP primer and probe of COL4A1/COL4A2:
   SEQ ID NO.7: COL4A1/COL4A2-FP: 5'-AGAGAGTTTAGTAAGGTCGGGC-3'
   SEQ ID NO.8: COL4A1/COL4A2-RP: 5'-GACTTCAAAAACTACTACCCG-3' or
   SEQ ID NO.14: COL4A1/COL4A2-FP:
      5'-AGAGAGTTTAGTAAGGTCGGAC-3'
   SEQ ID NO.15: COL4A1/COL4A2-RP:
      5'-GACTTCAAAAACTACTACCCG-3'
   SEQ ID NO.9: COL4A1/COL4A2-Probe: 5'-TGTCGGTGTGTCGTCGGC-3'

The two pairs of primers of COL4A1/COL4A2 have the same amplification specificity and can distinguish positive and negative samples well, and either one can be selected.
SEQ ID NO.10: capture sequence of ITGA4:
   5'-CTACGCGCGGCTGCAGGGGGCGCTGGGGAACCT-3'
qMSP primer and probe of ITGA4:
   SEQ ID NO.11: ITGA4-FP: 5'-ACGCGAGTTTTGCGTAGAC-3'
   SEQ ID NO.12:ITGA4-RP: 5'-GCTAAATAAAATCCCGAACG-3' or
   SEQ ID NO.16: ITGA4-FP: 5'-ACGCGAGTTTTGCGTAGTC-3'
   SEQ ID NO.17: ITGA4-RP: 5'-GCTAAATAAAATCCCGAACG-3'
   SEQ ID NO.13: ITGA4-Probe: 5'-ACGGAGTTCGGTTTTGCGTTTTC-3'

The two pairs of primers of ITGA4 have the same amplification specificity and can distinguish positive and negative samples well, and either one can be selected.

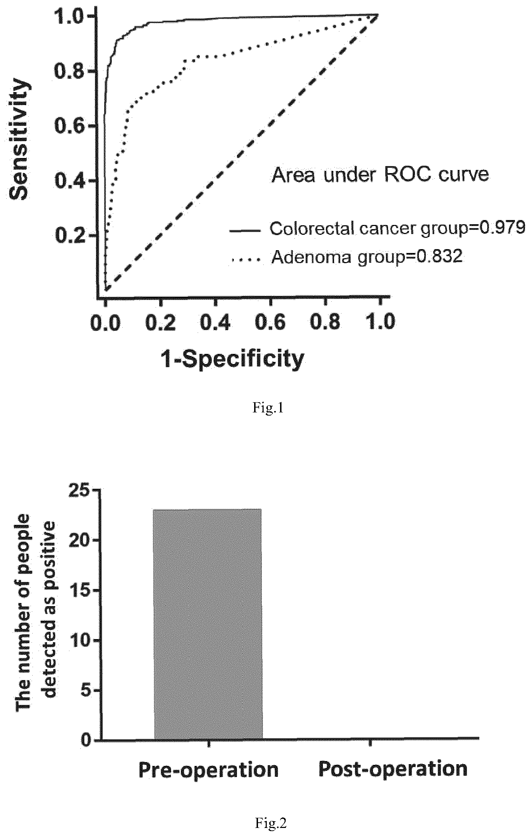

According to the results of these 935 fecal specimens, the ROC curve of the marker combination for colorectal cancer and adenoma detection is drawn using IBMSPSS statistics 20 software, as shown in FIG. 1.

The results show that the SDC2, COL4A1/COL4A2 and ITG4 gene combined methylation detection has a sensitivity of 91.36% for colorectal cancer and a sensitivity of 50.75% for adenoma when the specificity is 95.28%; and with the SDC2, COL4A1/COL4A2 and ITG4 gene combined methylation detection, the area under the ROC curve for colorectal cancer detection is 0.979, 95% CI is 0.970 to 0.988, the area under the ROC curve for adenoma detection is 0.832, and 95% CI is 0.771 to 0.894.

### Example 2

23 patients diagnosed with colorectal cancer were selected, fecal specimens of the patients were collected pre- and 3-6 months post-operation, the marker combination (SDC2, COL4A1/COL4A2 and ITGA4) of the present disclosure was used to detect 46 fecal specimens pre- and post-operation, and the methylation levels of the marker combination in the feces before and after operation were compared.

The detection process and result determination standard of the marker combination are the same as in Example 1. Target gene is determined as positive with a CT value ≤ 38, and as negative with a CT value > 38. The results are as shown in FIG. 2.

The results show that the detection results of these 23 patients with colorectal cancer are all positive before the operation. The fecal specimens are tested again after the operation, and the results are all negative. It indicates that the methylation level of the marker combination is significantly reduced after the tumor is excised, suggesting that the marker combination can be used for postoperative follow-up monitoring of patients with colorectal cancer.

### Comparative Example 1

The studies find that the detection of methylated SOX21 gene in feces can be used for auxiliary diagnosis of colorectal cancer, and the detection of colorectal cancer in feces with methylated SOX21 is now used as a comparative example. The following is the procedure and results of detecting SOX21 gene in feces.

240 fecal specimens (80 cases of colorectal cancer, 77 cases of adenoma (≥1 cm), 83 cases of normal, all confirmed by colonoscopy or pathology) were selected for grinding and centrifugation, 50 ul of capture magnetic beads (containing capture sequences of SOX21 and reference gene ACTB) were added, and the operations were carried out according to the technical solution described in Example 1. Finally, 20 ul of Bisulfite-transformed DNA was obtained. Then qMSP detection was carried out, and finally the methylation level of SOX21 in the specimen was determined according to the CT value.

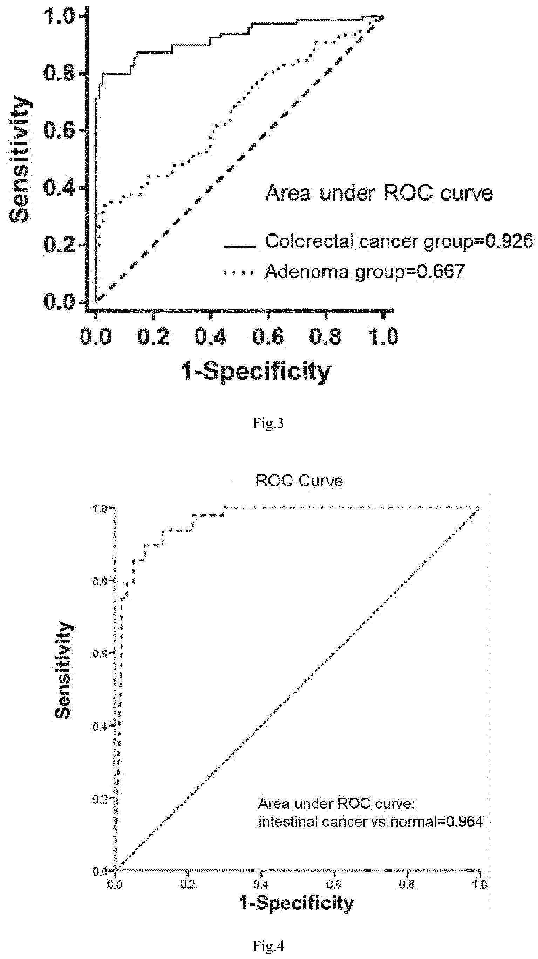

For the capture sequence, the primer and the probe contained in the reagent, and the qMSP reaction system and the procedures, see reference [2]. Based on the results of 240 fecal specimens, the ROC curve of SOX21 gene for colorectal cancer and adenoma detection is drawn, as shown in FIG. 3.

The results show that when the specificity of methylated SOX21 is 97.6%, the sensitivity to colorectal cancer is 80% and the sensitivity to adenoma is 33.8%; and the area under the ROC curve for SOX21 colorectal cancer detection is 0.926, 95% CI is 0.885 to 0.968, the area under the ROC curve for adenoma detection is 0.667, and 95% CI is 0.583 to 0.751.

### Comparative Example 2

161 fecal specimens (79 cases of colorectal cancer, 82 cases of normal, all confirmed by colonoscopy or pathology) were selected for grinding and centrifugation, 50 ul of capture magnetic beads (containing capture sequences of SOX21, SDC2 and reference gene ACTB) were added, and the operations were carried out according to the technical solution described in Example 1. Finally, 20 ul of Bisulfite-transformed DNA was obtained. Then qMSP detection was carried out, and finally the methylation levels of SOX21 and SDC2 in the specimen were determined according to the CT value.

The capture sequence, the primer and the probe of SOX21, and the qMSP reaction system and the procedures were the same as those in Comparative Example 1. The capture sequence, the primer and the probe of SDC2, and the qMSP reaction system and the procedures were the same as those in Example 1.

The detection results show that the methylated SDC2 gene detection has a sensitivity of 88.6%, and a specificity of 93.9%; and the area under the curve is 0.939. The methylated SOX21 gene has a sensitivity of 79.7% and a specificity of 86.6%; and the area under the curve is 0.924. The methylated SOX21 gene and methylated SDC2 gene combination has a detection sensitivity of 87.34% and a specificity of 93.9%. The detection sensitivity of the methylated SDC2 gene and methylated SOX21 gene combined detection is lower than that of the methylated SDC2 gene alone as a marker for colon cancer, and is also lower than that of the combined detection of SDC2, COL4A1/COL4A2 and ITG4 genes of the present disclosure.

### Comparative Example 3

109 fecal specimens (61 cases of colorectal cancer, and 48 cases of normal, all confirmed by colonoscopy or pathology) were selected for grinding and centrifugation, 50 ul of capture magnetic beads (containing capture sequences of ITGA4, COL4A1/COL4A2 and reference gene ACTB) were added.

The capture sequences, the primers and the probes of ITGA4, COL4A1/COL4A2, and the qMSP reaction system and the procedures were the same as in Example 1.

Based on the results of these 109 fecal specimens, the ROC curve of the marker combination for colorectal cancer detection is drawn, as shown in FIG. 4.

The results show that the area under the curve distinguishing colorectal cancer from normal human samples is 0.964. When the positive determination Ct value is 38, the methylated ITGA4+COL4A1/COL4A2 gene has a sensitivity of 86.89% and a specificity of 91.67% for colorectal cancer detection. Its sensitivity and specificity are both lower than the sensitivity and specificity of the SDC2, COL4A1/COL4A2 and ITG4 gene combined methylation detection of the present disclosure.

The descriptions above are only some implementations of the present disclosure. It should be pointed out that for a person of ordinary skill in the art, without departing from the principle of the present disclosure, several improvements and modifications can be made, and these improvements and modifications shall also fall within the protection scope of the present disclosure.

References:
1. Fischer G, Schmidt, Cornelia, et al. Identification of a novel sequence element in the common promoter region of human collagen type IV genes, involved in the regulation of divergent transcription. Biochem. J. 1993 292:687-695.
2. Niu F, Wen J, Fu X, et al. Stool DNA Test of Methylated Syndecan-2 for the Early Detection of Colorectal Neoplasia. Cancer Epidemiol Biomarkers Prev 2017; 26:1411-1419.
3. Liu Xianglin, Wen Jialing, Niu Feng, et al. Aberrant methylation of SOX21 gene as a potential marker for stool-based detection of colorectal cancer [J]. Chinese Journal of Cancer Prevention and Treatment ,2018(24):1710-1715.

## Claims

1. A gene marker combination, comprising SDC2, COL4A1/COL4A2 and ITGA4.

2. A detection reagent/detection kit, comprising a reagent for methylation detection of the SDC2, COL4A1/COL4A2 and ITGA4 genes, wherein:
preferably, the detection reagent/detection kit comprises a capture sequence, a primer and/or a probe for the SDC2, COL4A1/COL4A2 and ITGA4 genes;
preferably, the detection reagent/detection kit comprises a capture sequence, a primer and/or a probe obtainable for the CpG island of each gene;
preferably, the capture sequence for methylation detection of the SDC2 gene comprises any one of nucleotide sequences as shown below:
I. a nucleotide sequence having at least 85% or at least 90% or at least 91% or at least 92% or at least 93% or at least 94% or at least 95% or at least 96% or at least 97% or at least 98% or at least 99%, or 100% identity to the sequences as set forth in any of SEQ ID NO:1 and 2; and
II. a sequence complementary to the sequence as set forth in I; and/or
the capture sequence for methylation detection of the COL4A1/COL4A2 gene comprises any one of nucleotide sequences as shown below:
III. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:6; and
IV. a sequence complementary to the sequence as set forth in III; and/or
the capture sequence for methylation detection of the ITGA4 gene comprises any one of nucleotide sequences as shown below:
V. a nucleotide sequence having at least 85% or at least 90% or at least 91% or at least 92% or at least 93% or at least 94% or at least 95% or at least 96% or at least 97% or at least 98% or at least 99%, or 100% identity to a sequence as set forth in SEQ ID NO:10; and
VI. a sequence complementary to the sequence as set forth in V;
preferably, the upstream primer in the primer for methylation detection of the SDC2 gene comprises any one of nucleotide sequences as shown below:
VII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:3; and
VIII. a sequence complementary to the sequence as set forth in VII; and/or
the downstream primer in the primer for methylation detection of the SDC2 gene comprises any one of nucleotide sequences as shown below:
IX. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:4; and
X. a sequence complementary to the sequence as set forth in IX; and/or
the upstream primer in the primer for methylation detection of the COL4A1/COL4A2 gene comprises any one of nucleotide sequences as shown below:
XI. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:7 and 14; and
XII. a sequence complementary to the sequence as set forth in XI; and/or
the downstream primer in the primer for methylation detection of the COL4A1/COL4A2 gene comprises any one of nucleotide sequences as shown below:
XIII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:8 and 15; and
XIV. a sequence complementary to the sequence as set forth in XIII;
preferably, the primer pair for methylation detection of the COL4A1/COL4A2 gene is as set forth in SEQ ID NO:7 and SEQ ID NO:8; and
preferably, the primer pair for methylation detection of the COL4A1/COL4A2 gene is as set forth in SEQ ID NO:14 and SEQ ID NO:15; and/or
the upstream primer in the primer for methylation detection of the ITGA4 gene comprises any one of nucleotide sequences as shown below:
XV. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:11 and SEQ ID NO:16; and
XVI. a sequence complementary to the sequence as set forth in XV; and/or
the downstream primer in the primer for methylation detection of the ITGA4 gene comprises any one of nucleotide sequences as shown below:
XVII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in any of SEQ ID NO:12 and SEQ ID NO:17; and
XVIII. a sequence complementary to the sequence as set forth in XVII;
preferably, the primer pair for methylation detection of the ITGA4 gene is as set forth in SEQ ID NO:11 and SEQ ID NO:12.
preferably, the primer pair for methylation detection of the ITGA4 gene is as set forth in SEQ ID NO:16 and SEQ ID NO:17.
Preferably, the probe for methylation detection of the SDC2 gene comprises any one of nucleotide sequences as shown below:
XIX. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:5; and
XX. a sequence complementary to the sequence as set forth in XIX; and/or
the probe for methylation detection of the COL4A1/COL4A2 gene comprises any one of nucleotide sequences as shown below:
XXI. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:9; and
XXII. a sequence complementary to the sequence as set forth in XXI; and/or
the probe for methylation detection of the ITGA4 gene comprises any one of nucleotide sequences as shown below:
XXIII. a nucleotide sequence having at least 85% or at least 90% or at least 91% or at least 92% or at least 93% or at least 94% or at least 95% or at least 96% or at least 97% or at least 98% or at least 99%, or 100% identity to the sequence as set forth in SEQ ID NO:13; and
XXIV. a sequence complementary to the sequence as set forth in XXIII.

3. Use of a combined detection reagent for methylation of multiple genes in the preparation of a colorectal tumor detection reagent or kit, wherein the genes comprise SDC2, COL4A1/COL4A2 and ITGA4;
preferably, the combined detection reagent for methylation of multiple genes comprises a capture sequence, a primer and/or a probe for methylation detection of each gene;
preferably, the reagent comprises a capture sequence, a primer and/or a probe obtainable for the CpG island of each gene;
preferably, wherein the capture sequence for methylation detection of the SDC2 gene comprises any one of nucleotide sequences as shown below:
I. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:1 and 2; and
II. a sequence complementary to the sequence as set forth in I; and/or
the capture sequence for methylation detection of the COL4A1/COL4A2 gene comprises any one of nucleotide sequences as shown below:
III. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:6; and
IV. a sequence complementary to the sequence as set forth in III; and/or
the capture sequence for methylation detection of the ITGA4 gene comprises any one of nucleotide sequences as shown below:
V. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:10; and
VI. a sequence complementary to the sequence as set forth in V;
preferably, the upstream primer in the primer for methylation detection of the SDC2 gene comprises any one of nucleotide sequences as shown below:
VII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:3; and
VIII. a sequence complementary to the sequence as set forth in VII; and/or
the downstream primer in the primer for methylation detection of the SDC2 gene comprises any one of nucleotide sequences as shown below:
IX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:4; and
X. a sequence complementary to the sequence as set forth in IX; and/or
the upstream primer in the primer for methylation detection of the COL4A1/COL4A2 gene comprises any one of nucleotide sequences as shown below:
XI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:7 and 14; and
XII. a sequence complementary to the sequence as set forth in XI; and/or
the downstream primer in the primer for methylation detection of the COL4A1/COL4A2 gene comprises any one of nucleotide sequences as shown below:
XIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:8 and 15; and
XIV. a sequence complementary to the sequence as set forth in XIII;
preferably, the primer pair for methylation detection of the COL4A1/COL4A2 gene is as set forth in SEQ ID NO:7 and SEQ ID NO:8; and
preferably, the primer pair for methylation detection of the COL4A1/COL4A2 gene is as set forth in SEQ ID NO:14 and SEQ ID NO:15; and/or
the upstream primer in the primer for methylation detection of the ITGA4 gene comprises any one of nucleotide sequences as shown below:
XV. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:11 and SEQ ID NO:16; and
XVI. a sequence complementary to the sequence as set forth in XV; and/or
the downstream primer in the primer for methylation detection of the ITGA4 gene comprises any one of nucleotide sequences as shown below:
XVII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:12 and SEQ ID NO:17; and
XVIII. a sequence complementary to the sequence as set forth in XVII;
preferably, the primer pair for methylation detection of the ITGA4 gene is as set forth in SEQ ID NO:11 and SEQ ID NO:12.
preferably, the primer pair for methylation detection of the ITGA4 gene is as set forth in SEQ ID NO:16 and SEQ ID NO:17.
preferably, the probe for methylation detection of the SDC2 gene comprises any one of nucleotide sequences as shown below:
XIX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:5; and
XX. a sequence complementary to the sequence as set forth in XIX; and/or
the probe for methylation detection of the COL4A1/COL4A2 gene comprises any one of nucleotide sequences as shown below:
XXI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:9; and
XXII. a sequence complementary to the sequence as set forth in XXI; and/or
the probe for methylation detection of the ITGA4 gene comprises any one of nucleotide sequences as shown below:
XXIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:13; and
XXIV. a sequence complementary to the sequence as set forth in XXIII.

4. The use according to any one of claims 2-3, wherein the colorectal tumor is colorectal cancer or adenoma.

5. The use according to any one of claims 2-3, wherein a test sample for the detection reagent comprises tissue, body fluid or excrement;
preferably, the tissue comprises intestinal tissue;
preferably, the body fluid comprises blood, extracellular fluid, tissue fluid, lymphatic fluid,
cerebrospinal fluid or aqueous humor;
preferably, the blood comprises serum and plasma;
preferably, the excrement comprises sputum, urine, saliva or feces;
preferably, the excrement comprises feces.

6. A colorectal tumor detection system, comprising the following components:
(1) a combined detection component for methylation of SDC2, COL4A1/COL4A2 and ITGA4 genes;
(2) a data processing component; and
(3) a result output component, wherein:
preferably, the methylation detection component comprises a methylation detection instrument;
preferably, the methylation detection instrument comprises one or more of a fluorescent quantitative PCR instrument, a PCR instrument, and a sequencer;
preferably, the data processing component comprises a data processing instrument;
preferably, the data processing instrument comprises one or more of a calculator and a computer;
preferably, the computer comprises a computer loaded with one or more software of SPSS, SAS, and Excel;
preferably, the result output component comprises a result output device;
preferably, the result output device comprises one or more of a screen and a paper report;
preferably, the methylation detection component further comprises the combined detection reagent for methylation of multiple genes according to any one of claims 3-4;
preferably, the data processing component is configured to a. receive the test data of a test sample and a normal control sample; b. store the test data of the test sample and the normal control sample; c. compare the test data of the test sample with that of the normal control sample having the same type; and d. respond to the probability or possibility that a subject will develop a colorectal tumor according to the comparison results; and
preferably, the result output component is used to output the probability or possibility that a subject will develop a colorectal tumor.

7. The colorectal tumor detection system according to claim 6, wherein the determination standard of the data processing component is to determine colorectal tumor specimens and normal specimens according to the cutoff of the Ct value of the fluorescence quantitative level for methylation;
preferably, the cutoff of the Ct value ranges from about 36 to 39; more preferably, the cutoff of the Ct value is about 38; and
preferably, when the Ct value of the test sample is less than the cutoff of the Ct value, it is determined as the colorectal tumor sample, and when the Ct value of the test sample is greater than or equal to the cutoff of the Ct value, it is determined as the normal sample.

8. The colorectal tumor detection system according to any one of claims 6-7, wherein the colorectal tumor is colorectal cancer or adenoma.

9. The colorectal tumor detection system according to any one of claims 6-8, wherein the test sample for the detection system comprises tissue, body fluid or excrement;
preferably, the tissue is intestinal tissue;
preferably, the body fluid comprises blood, extracellular fluid, tissue fluid, lymphatic fluid, cerebrospinal fluid or aqueous humor;
preferably, the blood comprises serum and plasma;
preferably, the excrement comprises sputum, urine, saliva or feces; and
more preferably, the excrement is feces.

10. A method for diagnosing a colorectal tumor, comprising the following steps of:
(1) detecting the methylation levels of the SDC2, COL4A1/COL4A2 and ITGA4 genes in a test sample derived from the subject;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample, wherein:
preferably, methylation-specific quantitative PCR (qMSP) is used to detect the methylation levels of SDC2, COL4A1/COL4A2 and ITGA4 genes;
preferably, the colorectal tumor sample and the normal sample are determined according to the cutoff of the Ct value of the fluorescence quantitative level for methylation;
preferably, the cutoff of the Ct value ranges from about 36 to 39; more preferably, the cutoff of the Ct value is about 38;
preferably, when the Ct value of the test sample is less than the cutoff of the Ct value, it is determined as the colorectal tumor sample, and when the Ct value of the test sample is greater than or equal to the cutoff of the Ct value, it is determined as the normal specimen;
preferably, the test sample for the method comprises tissue, body fluid or excrement;
preferably, the tissue is intestinal tissue;
preferably, the body fluid comprises blood, extracellular fluid, tissue fluid, lymphatic fluid, cerebrospinal fluid or aqueous humor;
preferably, the blood comprises serum and plasma;
preferably, the excrement comprises sputum, urine, saliva or feces;
more preferably, the excrement is feces;
preferably, a capture sequence, a primer and/or a probe is used to detect the methylation levels of SDC2, COL4A1/COL4A2 and ITGA4 genes;
preferably, wherein the capture sequence for methylation detection of the SDC2 gene comprises any one of nucleotide sequences as shown below:
I. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:1 and 2; and
II. a sequence complementary to the sequence as set forth in I; and/or
wherein, the capture sequence for methylation detection of the COL4A1/COL4A2 gene comprises any one of nucleotide sequences as shown below:
III. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:6; and
IV. a sequence complementary to the sequence as set forth in III; and/or
wherein, the capture sequence for methylation detection of the ITGA4 gene comprises any one of nucleotide sequences as shown below:
V. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:10; and
VI. a sequence complementary to the sequence as set forth in V;
preferably, wherein the upstream primer in the primer for methylation detection of the SDC2 gene comprises any one of nucleotide sequences as shown below:
VII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:3; and
VIII. a sequence complementary to the sequence as set forth in VII; and/or
the downstream primer in the primer for methylation detection of the SDC2 gene comprises any one of nucleotide sequences as shown below:
IX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:4; and
X. a sequence complementary to the sequence as set forth in IX; and/or
wherein, the upstream primer in the primer for methylation detection of the COL4A1/COL4A2 gene comprises any one of nucleotide sequences as shown below:
XI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:7 and 14; and
XII. a sequence complementary to the sequence as set forth in XI; and/or
wherein, the downstream primer in the primer for methylation detection of the COL4A1/COL4A2 gene comprises any one of nucleotide sequences as shown below:
XIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:8 and 15; and
XIV. a sequence complementary to the sequence as set forth in XIII;
preferably, the primer pair for methylation detection of the COL4A1/COL4A2 gene is as set forth in SEQ ID NO:7 and SEQ ID NO:8; and
preferably, the primer pair for methylation detection of the COL4A1/COL4A2 gene is as set forth in SEQ ID NO:14 and SEQ ID NO:15; and/or
wherein, the upstream primer in the primer for methylation detection of the ITGA4 gene comprises any one of nucleotide sequences as shown below:
XV. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:11 and SEQ ID NO:16; and
XVI. a sequence complementary to the sequence as set forth in XV; and/or
wherein, the downstream primer in the primer for methylation detection of the ITGA4 gene comprises any one of nucleotide sequences as shown below:
XVII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:12 and SEQ ID NO:17; and
XVIII. a sequence complementary to the sequence as set forth in XVII;
preferably, the primer pair for methylation detection of the ITGA4 gene is as set forth in SEQ ID NO:11 and SEQ ID NO:12.
preferably, the primer pair for methylation detection of the ITGA4 gene is as set forth in SEQ ID NO:16 and SEQ ID NO:17.
preferably, wherein the probe for methylation detection of the SDC2 gene comprises any one of nucleotide sequences as shown below:
XIX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:5; and
XX. a sequence complementary to the sequence as set forth in XIX; and/or
wherein, the probe for methylation detection of the COL4A1/COL4A2 gene comprises any one of nucleotide sequences as shown below:
XXI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:9; and
XXII. a sequence complementary to the sequence as set forth in XXI; and/or
wherein, the probe for methylation detection of the ITGA4 gene comprises any one of nucleotide sequences as shown below:
XXIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:13; and
XXIV. a sequence complementary to the sequence as set forth in XXIII.

11. A method for diagnosing a colorectal tumor, comprising the following steps of:
(1) detecting the methylation levels of the SDC2, COL4A1/COL4A2 and ITGA4 genes in a test sample derived from the subject; the detecting comprising contacting the test sample of the subject with a detection reagent for the methylation levels of SDC2, COL4A1/COL4A2 and ITGA4 genes;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample, wherein:
preferably, methylation-specific quantitative PCR (qMSP) is used to detect the methylation levels of SDC2, COL4A1/COL4A2 and ITGA4 genes;
preferably, the colorectal tumor sample and the normal sample are determined according to the cutoff of the Ct value of the fluorescence quantitative level for methylation;
preferably, the cutoff of the Ct value ranges from about 36 to 39; more preferably, the cutoff of the Ct value is about 38;
preferably, when the Ct value of the test sample is less than the cutoff of the Ct value, it is determined as the colorectal tumor sample, and when the Ct value of the test sample is greater than or equal to the cutoff of the Ct value, it is determined as the normal specimen;
preferably, the test sample for the method comprises tissue, body fluid or excrement;
preferably, the tissue is intestinal tissue;
preferably, the body fluid comprises blood, extracellular fluid, tissue fluid, lymphatic fluid,
cerebrospinal fluid or aqueous humor;
preferably, the blood comprises serum and plasma;
preferably, the excrement comprises sputum, urine, saliva or feces;
more preferably, the excrement is feces;
preferably, a capture sequence, a primer and/or a probe is used to detect the methylation levels of SDC2, COL4A1/COL4A2 and ITGA4 genes;
preferably, wherein the capture sequence for methylation detection of the SDC2 gene comprises any one of nucleotide sequences as shown below:
I. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:1 and 2; and
II. a sequence complementary to the sequence as set forth in I; and/or
wherein, the capture sequence for methylation detection of the COL4A1/COL4A2 gene comprises any one of nucleotide sequences as shown below:
III. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:6; and
IV. a sequence complementary to the sequence as set forth in III; and/or
wherein, the capture sequence for methylation detection of the ITGA4 gene comprises any one of nucleotide sequences as shown below:
V. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:10; and
VI. a sequence complementary to the sequence as set forth in V;
preferably, wherein the upstream primer in the primer for methylation detection of the SDC2 gene comprises any one of nucleotide sequences as shown below:
VII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:3; and
VIII. a sequence complementary to the sequence as set forth in VII; and/or
the downstream primer in the primer for methylation detection of the SDC2 gene comprises any one of nucleotide sequences as shown below:
IX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:4; and
X. a sequence complementary to the sequence as set forth in IX; and/or
wherein, the upstream primer in the primer for methylation detection of the COL4A1/COL4A2 gene comprises any one of nucleotide sequences as shown below:
XI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:7 and 14; and
XII. a sequence complementary to the sequence as set forth in XI; and/or
wherein, the downstream primer in the primer for methylation detection of the COL4A1/COL4A2 gene comprises any one of nucleotide sequences as shown below:
XIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:8 and 15; and
XIV. a sequence complementary to the sequence as set forth in XIII;
preferably, the primer pair for methylation detection of the COL4A1/COL4A2 gene is as set forth in SEQ ID NO:7 and SEQ ID NO:8; and
preferably, the primer pair for methylation detection of the COL4A1/COL4A2 gene is as set forth in SEQ ID NO:14 and SEQ ID NO:15; and/or
wherein, the upstream primer in the primer for methylation detection of the ITGA4 gene comprises any one of nucleotide sequences as shown below:
XV. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:11 and SEQ ID NO:16; and
XVI. a sequence complementary to the sequence as set forth in XV; and/or
wherein, the downstream primer in the primer for methylation detection of the ITGA4 gene comprises any one of nucleotide sequences as shown below:
XVII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:12 and SEQ ID NO:17; and
XVIII. a sequence complementary to the sequence as set forth in XVII;
preferably, the primer pair for methylation detection of the ITGA4 gene is as set forth in SEQ ID NO:11 and SEQ ID NO:12.
preferably, the primer pair for methylation detection of the ITGA4 gene is as set forth in SEQ ID NO:16 and SEQ ID NO:17.
preferably, wherein the probe for methylation detection of the SDC2 gene comprises any one of nucleotide sequences as shown below:
XIX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:5; and
XX. a sequence complementary to the sequence as set forth in XIX; and/or
wherein, the probe for methylation detection of the COL4A1/COL4A2 gene comprises any one of nucleotide sequences as shown below:
XXI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:9; and
XXII. a sequence complementary to the sequence as set forth in XXI; and/or
wherein, the probe for methylation detection of the ITGA4 gene comprises any one of nucleotide sequences as shown below:
XXIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:13; and
XXIV. a sequence complementary to the sequence as set forth in XXIII.

12. A method for treating a colorectal tumor, comprising the following steps of:
(1) detecting the methylation levels of the SDC2, COL4A1/COL4A2 and ITGA4 genes in a test sample derived from the subject;
(2) comparing the methylation levels of the SDC2, COL4A1/COL4A2 and ITGA4 genes in the test sample with that in a normal control sample; and
(3) diagnosing a colorectal tumor based on the deviation of the methylation level of the test sample from that of the normal control sample; if the subject is diagnosed as a patient with colorectal tumor, administering to the subject a drug for treating the colorectal tumor, wherein:
preferably, methylation-specific quantitative PCR (qMSP) is used to detect the methylation levels of SDC2, COL4A1/COL4A2 and ITGA4 genes;
preferably, the colorectal tumor sample and the normal sample are determined according to the cutoff of the Ct value of the fluorescence quantitative level for methylation;
preferably, the cutoff of the Ct value ranges from about 36 to 39; more preferably, the cutoff of the Ct value is about 38;
preferably, when the Ct value of the test sample is less than the cutoff of the Ct value, it is determined as the colorectal tumor sample, and when the Ct value of the test sample is greater than or equal to the cutoff of the Ct value, it is determined as the normal specimen;
preferably, the test sample for the method comprises tissue, body fluid or excrement;
preferably, the tissue is intestinal tissue;
preferably, the body fluid comprises blood, extracellular fluid, tissue fluid, lymphatic fluid,
cerebrospinal fluid or aqueous humor;
preferably, the blood comprises serum and plasma;
preferably, the excrement comprises sputum, urine, saliva or feces;
more preferably, the excrement is feces;
preferably, a capture sequence, a primer and/or a probe is used to detect the methylation levels of SDC2, COL4A1/COL4A2 and ITGA4 genes;
preferably, wherein the capture sequence for methylation detection of the SDC2 gene comprises any one of nucleotide sequences as shown below:
I. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:1 and 2; and
II. a sequence complementary to the sequence as set forth in I; and/or
wherein, the capture sequence for methylation detection of the COL4A1/COL4A2 gene comprises any one of nucleotide sequences as shown below:
III. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:6; and
IV. a sequence complementary to the sequence as set forth in III; and/or
wherein, the capture sequence for methylation detection of the ITGA4 gene comprises any one of nucleotide sequences as shown below:
V. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:10; and
VI. a sequence complementary to the sequence as set forth in V;
preferably, wherein the upstream primer in the primer for methylation detection of the SDC2 gene comprises any one of nucleotide sequences as shown below:
VII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:3; and
VIII. a sequence complementary to the sequence as set forth in VII; and/or
the downstream primer in the primer for methylation detection of the SDC2 gene comprises any one of nucleotide sequences as shown below:
IX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:4; and
X. a sequence complementary to the sequence as set forth in IX; and/or
wherein, the upstream primer in the primer for methylation detection of the COL4A1/COL4A2 gene comprises any one of nucleotide sequences as shown below:
XI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:7 and 14; and
XII. a sequence complementary to the sequence as set forth in XI; and/or
wherein, the downstream primer in the primer for methylation detection of the COL4A1/COL4A2 gene comprises any one of nucleotide sequences as shown below:
XIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:8 and 15; and
XIV. a sequence complementary to the sequence as set forth in XIII;
preferably, the primer pair for methylation detection of the COL4A1/COL4A2 gene is as set forth in SEQ ID NO:7 and SEQ ID NO:8; and
preferably, the primer pair for methylation detection of the COL4A1/COL4A2 gene is as set forth in SEQ ID NO:14 and SEQ ID NO:15; and/or
wherein, the upstream primer in the primer for methylation detection of the ITGA4 gene comprises any one of nucleotide sequences as shown below:
XV. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:11 and SEQ ID NO:16; and
XVI. a sequence complementary to the sequence as set forth in XV; and/or
wherein, the downstream primer in the primer for methylation detection of the ITGA4 gene comprises any one of nucleotide sequences as shown below:
XVII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:12 and SEQ ID NO:17; and
XVIII. a sequence complementary to the sequence as set forth in XVII;
preferably, the primer pair for methylation detection of the ITGA4 gene is as set forth in SEQ ID NO:11 and SEQ ID NO:12.
preferably, the primer pair for methylation detection of the ITGA4 gene is as set forth in SEQ ID NO:16 and SEQ ID NO:17.
preferably, wherein the probe for methylation detection of the SDC2 gene comprises any one of nucleotide sequences as shown below:
XIX. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:5; and
XX. a sequence complementary to the sequence as set forth in XIX; and/or
wherein, the probe for methylation detection of the COL4A1/COL4A2 gene comprises any one of nucleotide sequences as shown below:
XXI. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:9; and
XXII. a sequence complementary to the sequence as set forth in XXI; and/or
wherein, the probe for methylation detection of the ITGA4 gene comprises any one of nucleotide sequences as shown below:
XXIII. a nucleotide sequence having at least 85% or at least 90% or at least 91% or at least 92% or at least 93% or at least 94% or at least 95% or at least 96% or at least 97% or 98% or at least 99%, or 100% identity to the sequence as set forth in SEQ ID NO:13; and
XXIV. a sequence complementary to the sequence as set forth in XXIII.
